# EUROPEAN PATENT APPLICATION

(11) **EP 1 774 985 A1**
(43) Date of publication of application: **18.04.2007**
(21) Application number: 05765180.4
(22) Date of filing: 29.06.2005
(51) Int. Cl.: A61M 25/00

(54) **MEMBER FOR CATHETER'S POSITION VERIFICATION HAVING COLOR CHANGE INDICATOR AND CATHETER HAVING THE MEMBER FOR CATHETER'S POSITION VERIFICATION**

(30) Priority: 30.06.2004 JP 2004193069
(71) Applicant: JMS Co., Ltd., Hiroshima-shi, Hiroshima 730-8652 (JP)
(72) Inventor: FUJISHIMA, Ichiro, Seireimikatahara hospital, Hamamatu-shi, Shizuoka 433-8558 (JP); TOYOTA, Koichiro, JMS Co., Ltd., Hiroshima-shi, Hiroshima 730-8652 (JP); SAJIKI, Toshinobu, JMS Co., Ltd., Hiroshima-shi, Hiroshima 730-8652 (JP)
(74) Representative: Röthinger, Rainer
(86) International application number: PCT/JP2005/012004
(87) International publication number: WO 2006/003960

(57) **Abstract**

The present invention provides a member (2) for catheter position verification having permeability for body fluid, **characterized by** including a color change indicator (2A) capable of color changing upon contact with body fluid, the color change indicator (2A) when used fitted to a medical catheter (3) to be incorporated in the body or a connection member connected to the medical catheter (3). Further, there is provided a medical catheter comprising this member for catheter position verification. The use of the above medical catheter including the member for catheter position verification realizes simpler, more reliable and safer execution of treatment and examination.

## Description

### Technical Field

The present invention relates to a member for verifying the position of a catheter to be inserted in the body of a patient, the member having an indicator that changes color; a catheter to be inserted in the body, the catheter including the member for verifying the position of the catheter; and a method for verifying the position of the catheter in the body.

### Background Art

When a catheter is inserted in the body, it is important to accurately and easily know the position of the catheter, in particular, the position of the distal end thereof, which is a problem to be solved in the medical field using catheters. In particular, in the case of a feeding catheter to be indwelled in a part of the alimentary tract, such as the stomach or intestines, in order to administer a nutritional supplement to a patient, it is very important to verify the distal end thereof. The reason for this is that if the nutritional supplement is administered to the patient with the feeding catheter being placed by mistake in one of the respiratory organs, such as the trachea or lungs, pneumonia, asphyxia, or the like may be caused, which sometimes leads to life-threatening conditions.

In order to overcome the problem described above, various technical means have been proposed. As one of such means, when a catheter is indwelled inside the stomach or the like, the following method for verifying the position of the catheter is employed. For example, the Japan Nursing Association issues the medical and nursing safety management information No. 8 "Prevention of accident caused by wrong insertion/wrong injection of transnasal feeding tube", which encourages verification of the position of the catheter each time using the following methods or the like during insertion of the feeding catheter and injection of a nutritional supplement or the like. More specifically, it is instructed to check (1) if the gastric juice (gastric content) can be sucked up, (2) if a bubbling sound can be heard, etc. If verification is not possible by these methods, it is instructed to perform verification using radiography or to check if the sucked-up gastric content is acidic on litmus paper separately prepared. However, in the method for verifying the position using radiography, there are concerns that the QOL of the patient, in particular, the infant/child patient, might be degraded by exposure, and thus this method is hardly suitable for regular verification. Furthermore, in the verification method using the litmus paper, there are problems concerning preparation of the litmus paper separately, detachment of a syringe that has sucked up the gastric juice and addition of the sucked-up content dropwise onto the litmus paper. Thus, this method actually tends to be avoided in medical facilities.

In order to overcome such problems, methods for verifying the position of a catheter have been proposed in which the catheter is allowed to have a special function. In one of the methods, a radioactive substance is disposed at the tip of a catheter to be indwelled in the body, and by detecting the radiation emitted from the substance with an external detector, the tip of the catheter is detected and determined (Patent Document 1). However, in this method, since a radioactive metal is used, the health of the patient and the health of the practitioner are adversely affected, which is a problem. Another method for verifying the position of the tip of a catheter has also been proposed, in which a special catheter, at the tip of which a permanent magnet is disposed (Patent Document 2) and an apparatus for detecting the position of the tip of the special catheter (Patent Document 3) are combined. However, in this method for verifying the position of the tip of the catheter, since a special catheter is used, it is not possible to use a commonly used catheter. Furthermore, it is also necessary to use a special detection apparatus for verifying the position of the tip of the catheter. Thus, the implementation thereof increases the cost.

Patent Document 1: U.S. Patent No. 5,099,845
Patent Document 2: PCT Japanese Translation Patent Publication No. 2000-512873
Patent Document 3: PCT Japanese Translation Patent Publication No. 9-503054

### Disclosure of Invention

### Problems to be Solved by the Invention

In view of the above description, it is a major object of the present invention to provide a member for catheter position verification in which the position of a catheter inserted in the body of a patient, in particular, the position of the distal end region of a feeding catheter, during indwelling of the catheter inside the gastric tube or the like, can be verified more simply, reliably, and safely; and a catheter including the member for catheter position verification.

### Means for Solving the Problems

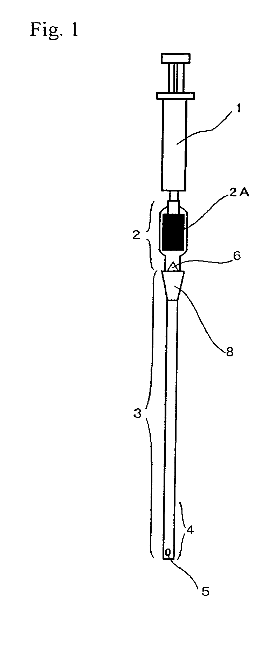

In order to solve the problems described above, the present inventors have conducted intensive research, and as a result, have conceived a member for catheter position verification which is used for a catheter to be inserted in the body and which includes an indicator that changes color upon contact with a body fluid, such as gastric juice (hereinafter referred to as the "color change indicator"). That is, the present invention relates to a member for catheter position verification which is a member fitted to a medial catheter to be inserted in the body, for example, joined to any visible section of the medical catheter or incorporated so as to be inserted into the catheter, when used, and which includes an indicator that changes color upon contact with body fluid. In the present invention, the change in color refers to the change in tone and/or color. Furthermore, the catheter of the present invention refers to a hollow (tubular) catheter that is inserted into the body. The catheter may have a catheter-connecting portion as shown in Fig. 1.

Furthermore, the present invention relates to a medical catheter which includes the member for catheter position verification described above, which is inserted in the body, and which is intended to perform various types of treatment in the body. The member for catheter position verification and a catheter constituting the medical catheter may be joined to each other either in a detachable manner or in an undetachable manner.
Furthermore, the present invention relates to a method for verifying the position of a catheter, the method using the catheter and including bringing the member for catheter position verification into contact with a body fluid, and visually recognizing the presence or absence of a color change, or a change, of the indicator to verify the position of the catheter in the body, for example, the position of the distal end of the catheter. According to this method for catheter verification, since the position of the catheter can be verified by bringing the member for catheter position verification into contact with a body fluid, and visually recognizing the presence or absence of a color change of the indicator, it is possible to verify the position of the catheter easily, reliably, and safely, and the problems associated with the conventional techniques can be solved, in particular, the time and labor associated with the litmus paper test in the gastric juice suction process.

The present invention will be described in more details below.
The color change indicator constituting the member for catheter position verification of the present invention has a pigment and a pigment carrier as basic components. When the color change indicator can be formed using a pigment alone, the color change indicator may have a pigment alone. The type of color change indicator used is not particularly limited as long as color change occurs upon contact with a body fluid to such a degree that the position of the catheter can be verified by visual observation. Since an acidic or alkaline body fluid (secretion) is often secreted, in particular, preferably, the color change indicator changes color depending on the hydrogen-ion concentration of a body fluid. For example, when the intended position of the catheter is the stomach, a color change indicator that changes color upon contact with an acidic secretion, such as gastric juice, may be used. Furthermore, in order to facilitate visual recognition of the color change upon contact with the body fluid, preferably, the color change indicator is adjusted to be neutral, i.e., to a pH of 6.5 to 7.5, before contact with the body fluid. Furthermore, if the color change indicator is adjusted to be neutral prior to contact with the body fluid, by the selection of an indicator or by the use of many types of indicator, it may be possible to use the indicator for detecting both an acidic secretion, such as gastric juice, and an alkaline secretion, such as duodenal juice. Thus, it is possible to verify the position of the catheter, for example, whether the distal end of the catheter is in the stomach or in the vicinity of the duodenum. As described above, for example, if color change occurs upon contact with gastric juice which is acidic, arrival of the catheter at the stomach can be verified. When the catheter is intended to be further inserted into the duodenum or the jejunum and indwelled therein, after the verification of the arrival at the inside of the stomach, by verifying no color change upon contact with the duodenal secretion or jejunal secretion or a color change that is different from the color change upon contact with the gastric juice, it is possible to confirm that the distal end of the catheter has passed through the stomach and reached the inside of the duodenum or jejunum.

Particularly preferably, the color change indicator is composed of a pigment that significantly changes color preferably under a pH of 6.0 or less, more preferably under a pH of 3.0 or less, because the arrival of the catheter at the inside of the stomach can be easily verified by the color change thereof. The color change of the pigment is thought to be caused by a change in the chemical structure depending on the pH (reversible change in color in the chemical equilibrium state), an alteration or decomposition of the chemical structure depending on the pH (irreversible change in color), or the like. The color change may be caused by any of the reasons described above. The type of pigment used is not particularly limited as long as the pigment is capable of constituting the color change indicator so that color change occurs upon contact with a body fluid to such a degree that the position of the catheter can be verified by visual observation as described above. In a structure in which the color change indicator is used so as to be inserted in the body, in view of safety, a food pigment is particularly preferable. Furthermore, food pigments can be broadly classified into natural food pigments and synthetic food pigments. Either type of pigment can be used as long as the problems of the present invention are satisfactorily solved.

Examples of natural food pigments include flavonoid pigments, carotenoid pigments, quinone pigments, and porphyrin pigments. Among these, anthocyanin pigments, which are flavonoid pigments, have a very high sensitivity to the pH and assume different colors at various pHs (i.e., the changes from acidity to neutrality toward alkalinity cause changes in color, such as red or reddish purple to purple or bluish purple to blue or green). Thus, anthocyanin pigments are considered to be pigments that cause a suitable change in color in order to solve the problems of the present invention. Furthermore, since anthocyanin pigments are polyphenols, their social image is highly favorable in consideration of the health-consciousness trend in recent years. Specific examples of anthocyanin pigments include red cabbage pigment, perilla pigment, purple corn pigment, purple potato pigment, red radish pigment, grape skin pigment, grape juice pigment, blueberry pigment, elderberry pigment, boysenberry pigment, and hibiscus pigment.

Anthocyanin pigments can be purchased from pigment manufacturers either in the form of powder or aqueous solution. However, because of the manufacturing process, available anthocyanin pigments are often acidic (at a pH of about 3.0). Therefore, when one of these anthocyanin pigments is mixed with a pigment carrier, it is necessary to neutralize the pigment in advance. The neutralization can be performed using an alkaline aqueous solution. Examples of the alkaline agent include hydroxide salts, such as sodium hydroxide, potassium hydroxide, calcium hydroxide, and magnesium hydroxide, and aqua ammonia. It is known that anthocyanin pigments become chemically unstable in a neutral to alkaline aqueous solution, and in addition to color change reaction caused by the change in the structure due to chemical equilibrium, decomposition of the chemical structure also occurs, resulting in decay in color, i.e., discoloration. However, in the present invention, it is confirmed that when a method is employed in which, immediately after the pigment and the pigment carrier are mixed to prepare a color change indicator precursor, the color change indicator precursor is dried, and the resulting product is used as a color change indicator (dry product), the stability of the pigment is maintained for a long time.

Other examples of natural food pigments include quinone pigments. Quinone pigments can be further classified into benzoquinone pigments, naphthoquinone pigments, anthraquinone pigments, phenanthrenequinone pigments, etc. Examples of anthraquinone pigments include cochineal pigment, lac pigment, and madder pigment. The cochineal pigment is a particularly suitable pigment in view of the abundance of safety data related to it and in view of the familiarity with this pigment. Cochineal pigment is sold in the form of powder, and an aqueous solution thereof is neutral. Therefore, the neutralization step associated with the use of anthocyanin pigments is not required, which is advantageous. Other examples of the pigment include synthetic food pigments. Synthetic food pigments can be further classified into food reds, food yellows, food blues, food greens, etc. Food reds are particularly suitable for color change of the color change indicator at a pH of 3.0 or less. Specific examples of food reds include Food Red No. 3 and Food Red No. 105. The change in color tone of the food reds is caused by the alteration or decomposition of the chemical structure depending on the pH (irreversible change in color tone). Although the food reds are disadvantageous in that the pH application range is a pH of 3.0 or less compared with the natural food pigments, the change in color tone occurs dramatically and/or definitely compared with natural food pigments, and thus contact or noncontact with an acid can be determined easily.

The pigment carrier used in the present invention is not particularly limited as long as it can carry the pigment stably and it is harmless to the living body. Preferred examples of the pigment carrier include fibers, a structure composed of the fibers, sponge, gel, and dietary fibers.
For the same reason as that in the selection of the pigment, from the safety point of view, the pigment carrier is preferably composed of a material that is safe even if ingested orally. More specifically, a material that is approved as food is preferable. Examples of materials that satisfy these conditions include agar, gelatin, alginic acid, cellulose, and starch. Among these, water-soluble materials are preferable. That is, with respect to a color change indicator composed of a water-soluble material that is safe even if ingested orally, after it is verified by the color change that a catheter to be inserted in the body has reached a desired position in the body, by dissolving the color change indicator, without detaching the color change indicator from the catheter, immediately, an intended medical action can be performed, for example, a nutritional supplement can be injected when the catheter is an enteral feeding catheter. Thus, the medical step can be simplified, which is advantageous.

The color change indicator of the present invention, which is composed of the pigment and the pigment carrier, is preferably a dry product. The main reason for this is that the liquid-absorbing ability of the color change indicator is improved by drying, and thus the color change of the color change indicator can be achieved in a short time compared with the use of a non-dried color change indicator. Furthermore, in the case where a pigment that is in a solution state and unstable is used, the dry color change indicator has the effect of stabilizing the pigment. For these reasons, not only the pigment carrier but also the pigment is desirably a dry product.
The drying method used for the drying is not particularly limited and can be a commonly known method, such as room-temperature drying, hot-air drying, or reduced-pressure drying, that can improve the liquid-absorbing ability of the color change indicator. However, when a pigment that is in a solution state and unstable is used, since discoloration due to the decomposition of the pigment is accelerated by heat, it is necessary to use a method in which drying is performed at a lower temperature and the color tone of the pigment can be maintained. For example, a freeze-drying method is a particularly suitable drying method that satisfies the conditions described above.
The structure of the pigment and/or the pigment carrier may have a combination in which one type is selected from the variety of components described above for each of the pigment and the pigment carrier in order to take advantage of the individual characteristics, a combination in which one type is selected for one of the pigment and the pigment carrier and two or more types are selected for the other, or a combination in which two or more types are selected for each of the pigment and the pigment carrier.

The color change indicator is, for example, incorporated into a tubular container to constitute a member for catheter position verification. The member for catheter position verification is connected to or incorporated into a medical catheter to be inserted in the body or a member connected to the catheter, such as a connecting tube, when used. In the member for catheter position verification, when color change is caused by contact with a body fluid, it is important that use and verification of the catheter position by color change can be performed in the connected or incorporated state without an extra operation other than a series of operations. That is, by using the member for catheter position verification that satisfies the requirements described above, the time and labor for verifying the position of a catheter for indwelling in the body is reduced. Furthermore, as described above, the color change indicator of the present invention is usually incorporated into a container member and then fitted to a catheter member. The color change indicator may be used in an embodiment in which the color change indicator is combined by kneading with the material for producing the catheter or in which the color change indicator is allowed to be present on the surface of the catheter.

In this description, in the embodiments and examples described below, an enteral feeding catheter will be described as the catheter to or into which the member for catheter position verification is connected or incorporated. In the catheter (for enteral feeding) to or into which the member for catheter position verification is connected or incorporated, it is possible to verify the arrival of the distal end region of the catheter indwelled in the body at a desired position inside the alimentary organ easily, accurately, and highly safely, thus being very useful. However, the application of the member for catheter position verification of the present invention is not limited to the enteral feeding catheter. The member for catheter position verification can also be connected to or incorporated into many known medical catheters which are intended to perform various types of treatment in the body, and it is possible to constitute medical catheters in which the position of the catheters can be verified easily, accurately, and safely.
In the present invention, the position of a catheter refers to the position of a portion of the catheter which is required to be verified in the body, and in many cases, the position of a distal end of the catheter. Alternatively, the position of the catheter may be the position of another portion or the position of the entire catheter. The expression "the distal end of the catheter" refers to the end itself on the living body side and in the vicinity of the end.

The embodiments of the present invention will be described below.

### Embodiment 1

Description will be made with reference to Fig. 1. Fig. 1 shows a syringe 1 which is a member that sucks gastric juice, a tubular member 2 for catheter position verification having a color change indicator 2A, and a catheter (for enteral feeding) 3. The insertion operation for allowing a distal end 4 of the catheter (for enteral feeding) 3 to reach the inside of the stomach and the verification of the distal end 4 are performed as follows.

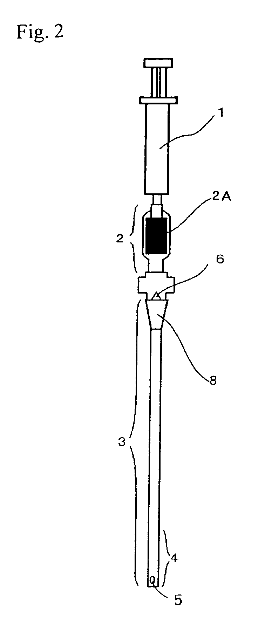

First, a marker is placed on the catheter (for enteral feeding) 3 in advance, and the approximate distance to the inside of the stomach is checked. Subsequently, when insertion into the body is performed, the catheter (for enteral feeding) 3 is transnasally inserted using the marker as an index. At the stage where insertion is completed to the position at which the catheter is assumed to have reached the inside the stomach, using the suction member, i.e., the syringe 1, suction of gastric juice is performed through a hole 5 in the distal end of the catheter. During this operation, gastric juice passes through the tubular member 2 for catheter position verification. When color change of the color change indicator 2A is confirmed simultaneously or substantially simultaneously with the passing of gastric juice, it is verified that the sucked-up liquid is gastric juice. Thus, it is verified that indwelling of the distal end 4 of the catheter (for enteral feeding) 3 inside the stomach is achieved. In this embodiment, suction of gastric juice is performed. It is preferable to prevent the sucked-up gastric juice from reflux into the body from the standpoint of safety and hygiene. Consequently, in Fig. 1, a check valve 6, as a mechanism for preventing the reflux of gastric juice, is incorporated into the tubular member 2 for catheter position verification. The gastric juice reflux prevention mechanism may be incorporated into the tubular member 2 for catheter position verification as shown in Fig. 1, or may be independently provided between a catheter-connecting portion 8 and the tubular member 2 for catheter position verification as shown in Fig. 2.

### Embodiment 2

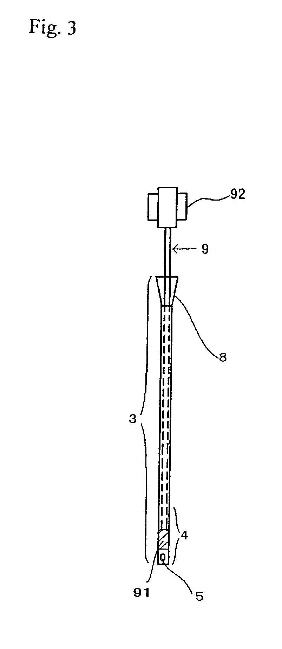

Description will be made with reference to Fig. 3. Fig. 3 shows a guidewire-like member 9 for catheter position verification having a color change indicator 91 and a catheter (for enteral feeding) 3. In this embodiment, the operation for allowing a distal end 4 of the catheter (for enteral feeding) 3 to reach the inside of the stomach and the verification of the distal end 4 are performed as follows.
The catheter (for enteral feeding) 3 into which the guidewire-like member 9 for catheter position verification having the color change indicator 91 is incorporated is allowed to reach the inside of the stomach as in Embodiment 1. Subsequently, in order to protrude the color change indicator 91 from a hole 5 in the distal end of the catheter and bring the color change indicator 91 into contact with gastric juice, a proximal portion 92 of the guidewire-like member for catheter position verification is pushed in. Subsequently, the guidewire-like member 9 for catheter position verification is slowly pulled out, and color change of the color change indicator 91 is checked. When color change is confirmed, it is verified that contact with gastric juice has occurred. Thus, it is verified that indwelling of the distal end 4 of the catheter (for enteral feeding) 3 inside the stomach is achieved. In the catheter position verification method in this embodiment, since suction of gastric juice is not required, a greater level of hygiene is obtained compared with the method according to Embodiment 1, and the burden to the patient is decreased. Furthermore, by selecting a material that has an excellent function as a guidewire for the body of the guidewire-like member 9 for catheter position verification, insertion of the catheter is facilitated, which is advantageous.

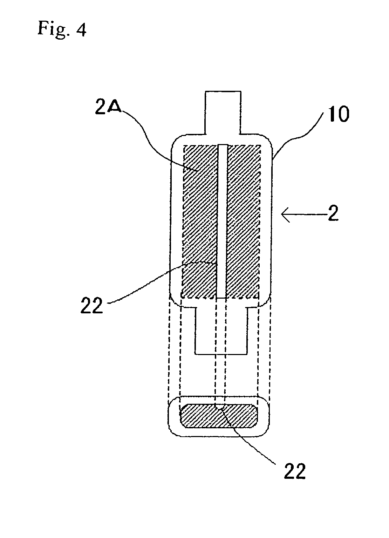

Figs. 4 to 9 each show a specific example of the member for catheter position verification. The color change indicators of these members for catheter position verification have a structure in which a body fluid sucked up through a suction means in the shape of a hole, such as a groove or a defective portion, easily passes (hereinafter referred to as the "easy flow structure").

### Embodiment 3

Fig. 4 shows a member 2 for catheter position verification including a color change indicator 2A placed in a tubular container 10, the color change indicator 2A having a groove 22 provided on one surface. A body fluid, sucked by a suction member, for example, a syringe shown in Fig. 1 or 2, passes through the groove 22 and is received into the suction member. The position of the catheter can be verified by the color change of the color change indicator 2A upon contact with the body fluid. Although not shown, the same effect can be expected in the case where grooves are provided on both surfaces, or the color change indicator has a cylindrical shape and one or more grooves are provided on the circumference thereof. Alternatively, instead of forming a groove on the surface of the color change indicator 2A, a groove may be formed on the inner surface of the tubular container.

### Embodiment 4

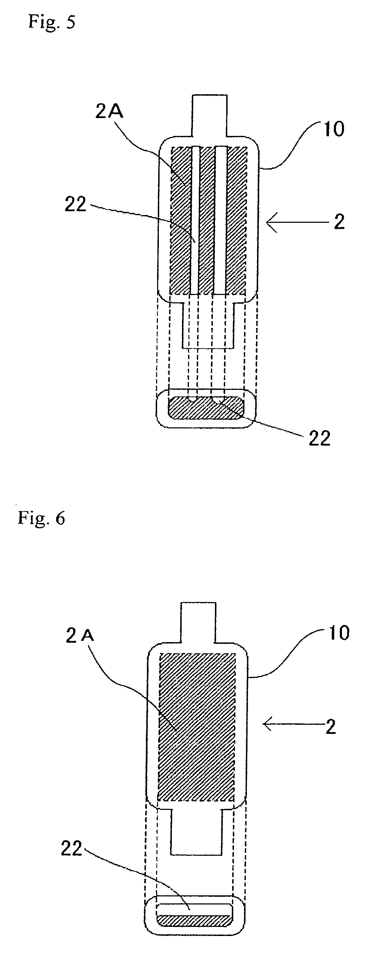

Fig. 5 shows a member 2 for catheter position verification which is the same as the member 2 for catheter position verification shown in Fig. 4 except that a plurality of grooves 22 are provided on one surface of the color change indicator 2A. Although the color change indicator 2A having two grooves 22 is shown in Fig. 5, the color change indicator may have more than two grooves on the surface thereof. Furthermore, in the color change indicator, besides the number of grooves, by changing the size, shape, or the like of the groove, a body fluid sucked by the suction member is allowed to pass through the member 2 for catheter position verification in various manners. For example, the body fluid may be allowed to flow only in a specific groove among a plurality of grooves and pass through the member 2 for catheter position verification. Furthermore, by using a soft material for the outer wall member so that the grooves have different liquid flow resistances, it is possible to allow a body fluid to flow in a predetermined groove. Thus, using one member for position verification, it is possible to perform body fluid suction for verifying the position of the catheter a plurality of times according to the number of grooves. Consequently, using one color change indicator, position verification can be performed a plurality of times.

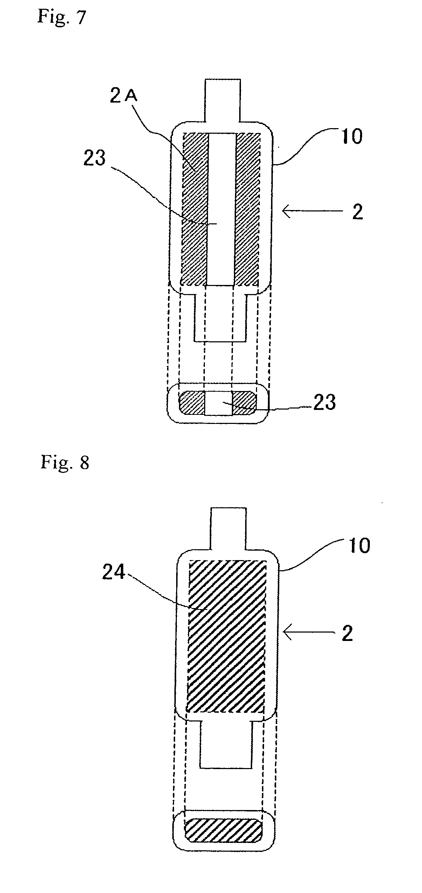

Fig. 6 shows a structure in which a groove 22 is large and the full face of one side is open (Embodiment 5). Fig. 7 shows a structure in which a color change indicator 2A is partially defective over a full thickness, i.e., divided into two or more (Embodiment 6). Fig. 7 shows a representative example in which one fractured portion 23 is present in the center. The shape of any of the color change indicators can solve the problems. Fig. 8 shows a color change indicator 2A which at least partially has a portion that allows a liquid to easily pass through, for example, a large porous structure 24, instead of the body fluid-passing portion, such as the groove or the defective portion (Embodiment 7).

### Embodiment 8

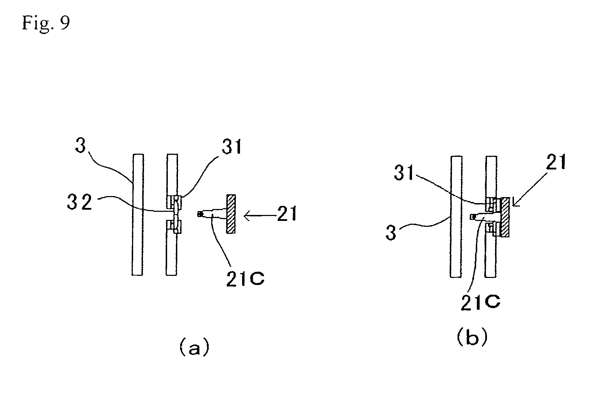

Fig. 9 shows a button-shaped member 21 for position verification and a feeding catheter 3 having a connecting portion 31, to which the button-shaped member 21 for position verification is connected, provided on the sidewall thereof.

That is, Fig. 9 shows the feeding catheter 3 and the button-shaped member 21 for position verification, the feeding catheter 3 having a connecting portion provided on the sidewall thereof, the connecting portion being composed of an elastic septum 32 having a slit, the button-shaped member 21 for position verification being capable of being connected to and disconnected from the connecting portion. The button-shaped member 21 for position verification includes a button-shaped container having a leg 21C, and a color change indicator 2A contained in the container.

Fig. 9(a) shows a state before the button-shaped member 21 for position verification is connected to the connecting portion 31, and Fig. 9(b) shows a state after the button-shaped member 21 for position verification is connected to the connecting portion 31.

In this embodiment, since the button-shaped member 21 for position verification can be easily connected to and detached from the connecting portion 31, position verification can be performed easily a plurality of times. Although the connecting portion 31 composed of the elastic septum 32 is formed in the feeding catheter 3 in this embodiment, the connecting portion 31 may be formed in the sidewall of a connecting tube (not shown) which is connected to the feeding catheter 3.

### Embodiment 9

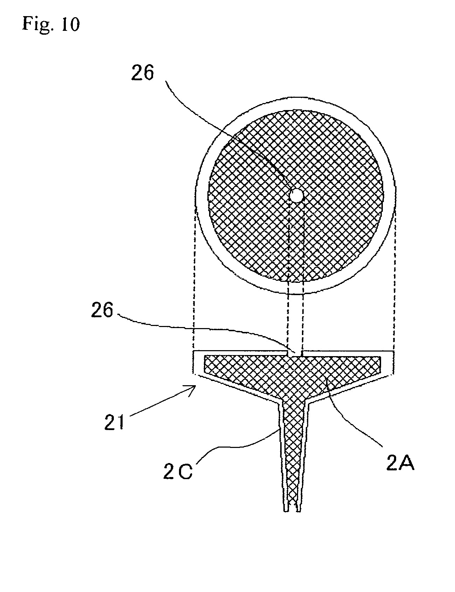

A button-shaped member 21 for position verification shown in Fig. 10 has a leg 2C which is inserted into and held by a connecting portion for the member, and is a hollow container-like structure. The button-shaped member 21 for position verification is provided with an air vent 26, and a hollow section thereof is filled with a color change indicator material 2A. Although the size of the vent 26 is not particularly limited, preferably, the vent 26 has a size to such an extent that does not allow the color change indicator material 2A to be dislodged when a body fluid is introduced into the hollow section. Similarly, although the size and shape of the indicator material 21 are not particularly limited, preferably, the indicator material 2A has a size and shape that prevent the indicator material 2A from being dislodged through the vent 26 during the introduction and discharge of a body fluid. Furthermore, the shape and/or the size of the button-shaped member for position verification are not necessarily limited to those shown in the drawing as long as the same function is accomplished.

### Embodiment 10

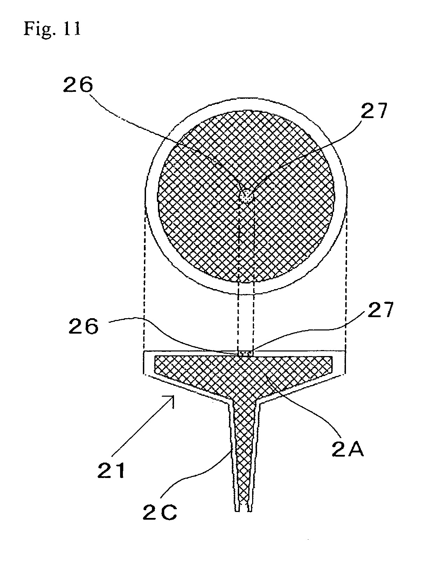

Fig. 11 shows a structure in which a hydrophobic porous filter 27 is fitted to the air vent 26 of the button-shaped member 21 for position verification shown Fig. 10. In the button-shaped member 21 for position verification shown in Fig. 10, when a body fluid is introduced in an amount larger than the volume of the hollow section, in some cases, the body fluid may partially leak from the air vent 26, resulting in a problem in view of safety and hygiene. In order to solve such a problem, in this embodiment, a hydrophobic porous filter 27 is fitted to the air vent 26, the hydrophobic porous filter 27 being capable of easily passing air but incapable of passing a liquid, such as water. Thus, leakage of the body fluid from the air vent 26 can be prevented.

### Embodiment 11

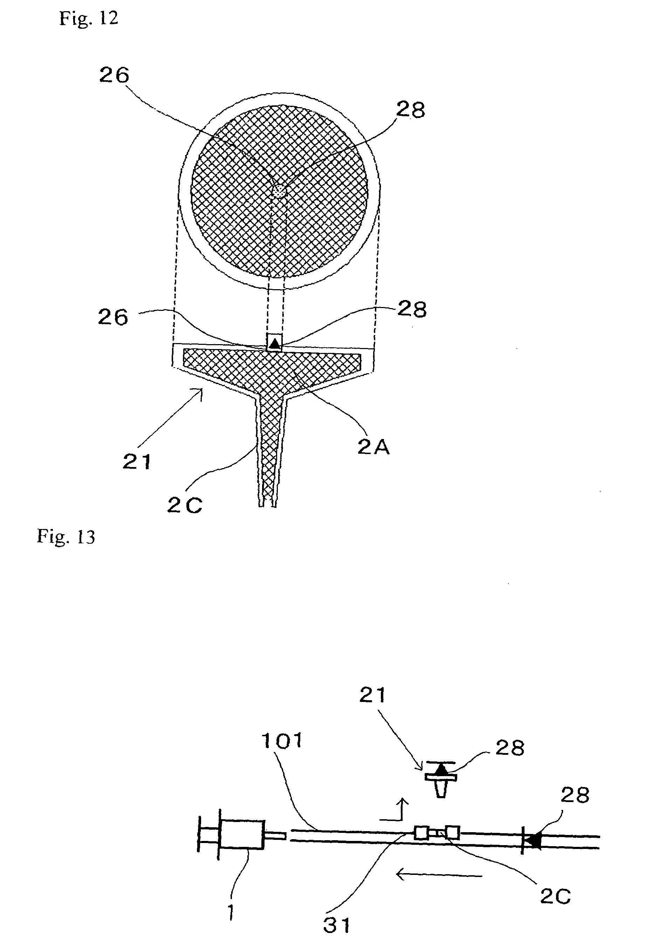

Fig. 12 shows a structure in which a check valve 28 is fitted to the air vent 26 shown in Fig. 10. Since the button-shaped member 21 for position verification shown in Fig. 10 or 11 has the air vent 26, it is not possible to suck a body fluid, such as gastric juice, with the member being fitted. Consequently, in order to impregnate the indicator material 21 with the body fluid, troublesome operations are required in which after the body fluid is sucked with a syringe or the like, the button-shaped member 21 for position verification is fitted, and the body fluid is introduced into the member for position verification. In order to simplify these operations, in this embodiment, the check valve 28 is fitted to the air vent. By providing the check valve 28, suction of the body fluid is possible not only before fitting but also after fitting of the button-shaped member 21 for position verification, and thus the operations are simplified.

### Embodiment 12

Fig. 13 shows a syringe 1, a member 21 for position verification, and a connecting tube 101 which connect between the syringe 1 and a feeding catheter 3 (not shown), the connecting tube 101 having a connecting portion 31 for the button-shaped member 21 for position verification. A check valve 28 is further provided in the connecting tube 101 at a position apart from the connecting portion 31 on the feeding catheter 3 side. Thus, by the action of the check valve 28 and another check valve 28 provided in the button-shaped member 21 for catheter position verification, the body fluid sucked into the syringe 1 can be impregnated into the button-shaped member 21 for position verification, without being returned to the catheter.

### Embodiment 12

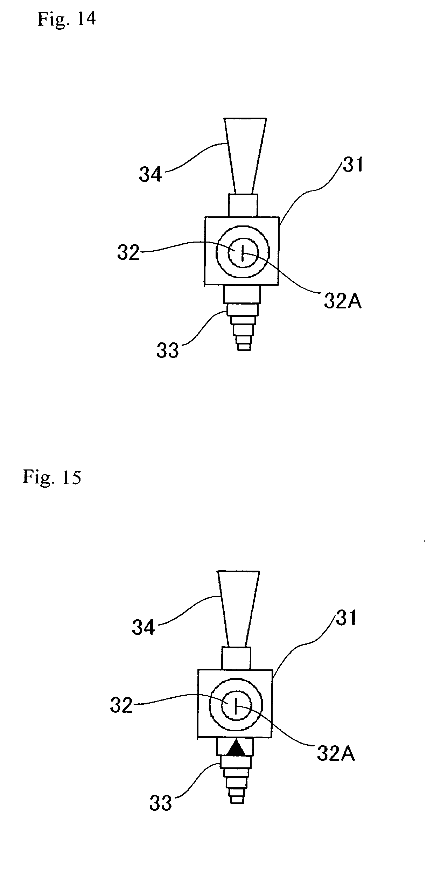

Fig. 14 shows an example of a connection member (connector) to be placed between a catheter, for example, a feeding catheter, and a connecting tube. The connector includes a connecting portion 31 for a member for catheter position verification, and a male connector 33 and a female connector 34 disposed in front and rear of the connecting portion 31. The connecting portion 31 for the member for catheter position verification is composed of a septum (elastic septum) 32 having a slit and/or a hole 32A. Consequently, the leg 2C of the button-shaped member 21 for position verification can be connected and disconnected a plurality of times. Fig. 15 shows an example in which a check valve 28 is further provided in the connection member 29 shown in Fig. 14. In this structure, a mechanism of preventing the sucked body fluid, such as gastric juice, from flowing back into the patient's body is added.

### Embodiment 13

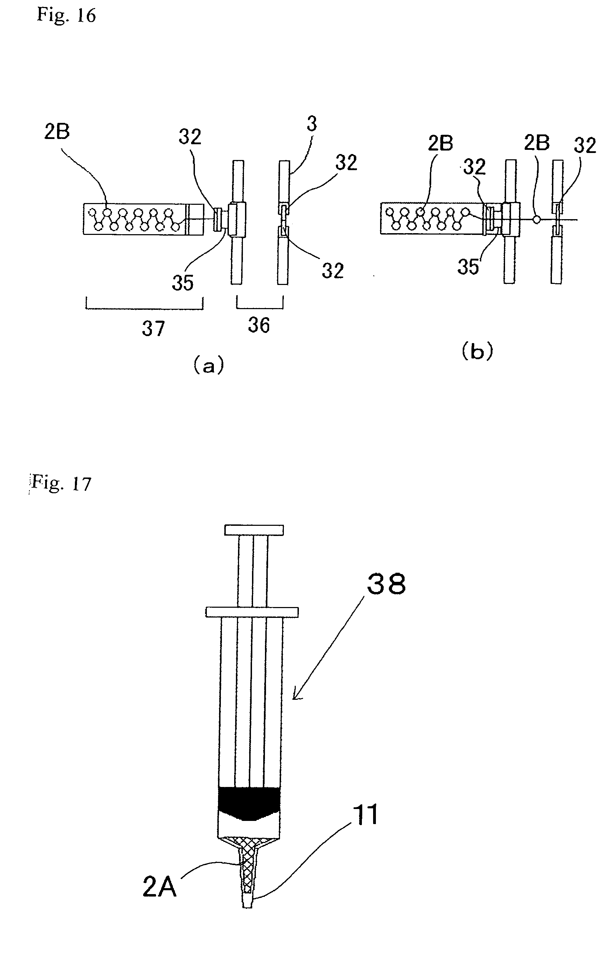

Fig. 16 shows an example of a structure in which position verification of a distal end of a catheter can be performed a plurality of times using a member for catheter position verification. The structure according to this embodiment can be used as a part of a feeding catheter or a connecting member for a feeding catheter. The structure in this example can be broadly divided into the following two portions: a portion in which a color change indicator material 2A is introduced into and discharged from a catheter while maintaining airtightness (hereinafter referred to as an "airtight portion 36") and a portion in which a plurality of pieces of the indicator material can be stored airtight (hereinafter referred to as a "storage portion 37"). The mechanism of introducing and discharging the member for position verification while maintaining airtightness can be realized by a connecting portion 31 having septum (elastic septum) 32 structures on facing sides of a catheter 3. The septum (elastic septum) 32 on the side of the catheter 3 into which the color change indicator material 2A is introduced is fitted to the catheter 3 with an adaptor 35. The storage portion 36 has a structure in which pieces of the indicator material 2A are joined to one another with a thread or the like, and by pulling one end of the thread, the pieces of the indicator material 2A can be pulled out continuously. The distance between the two adjacent pieces of the indicator material 2A is adjusted according to the distance between the opposing septa (elastic septa) 32. A member for joining the pieces of the color change indicator material 2A may be a material other than the thread as long as the intended purpose is accomplished, or may be a tube or the like having a hole at an appropriate position. Fig. 16(a) shows a state before introducing the color change indicator 2A into the catheter while maintaining airtightness, and Fig. 16(b) shows a state after introducing the color change indicator 2A into the catheter while maintaining airtightness.

### Embodiment 14

A member for catheter position verification shown in Fig. 17 includes a syringe 38 which is composed of a piston and a cylinder and which is a body fluid suction member, for example, used for suction of gastric juice. An indicator material 2A is placed in a cylinder tip 11 having a small diameter. A catheter, such as a feeding catheter, is connected to the cylinder tip 11 of the syringe 38. Although the form of the indicator material 2A is not particularly limited, the indicator material 2A preferably has a size and shape that prevent the indicator material 2A from easily flowing out during introduction and discharge of a body fluid into and from the body fluid suction member. Although the member for catheter position verification in this embodiment can verify the position of a catheter only once, the structure thereof is simple.

### Embodiment 15

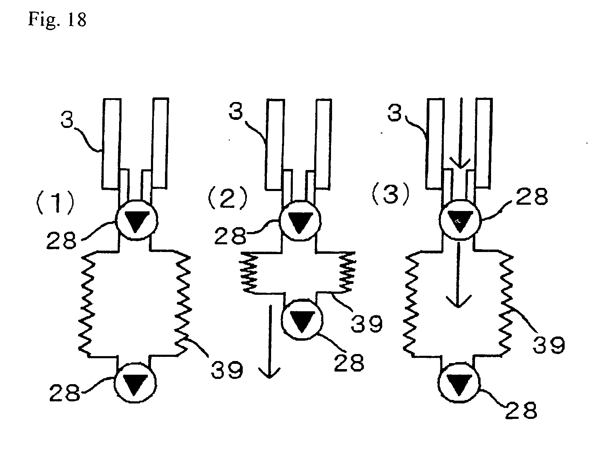

A member for catheter position verification shown in Fig. 18 includes a bellows-like body fluid suction member 39 in which an indicator material 2A (not shown) is placed and into which a body fluid, such as gastric juice, can be sucked. Preferably, a check valve 28 is provided between the bellows-like body fluid suction member 39 and a catheter 3, for example, a feeding catheter, the check valve 28 being capable of preventing a body fluid, such as gastric juice, sucked into the bellows-like body fluid suction member 39 from returning to the catheter. Furthermore, the check valve 28 may be provided in the bellows-like body fluid suction member 39 or the catheter 3. In the member for catheter position verification shown in Fig. 18, in addition to the check valve 28 on the catheter side, preferably, a check valve is also provided on the side opposite to the catheter connection side, the check valve being capable of discharging air from the bellows-like body fluid suction member 39, but preventing outside air from entering the bellows-like body fluid suction member 39. Fig. 18(1) shows a structure before use in this embodiment. Fig. 18(2) shows a structure in which the bellows portion of the bellows-like body fluid suction member 39 is contracted, and air in the bellows portion is discharged to outside in the direction indicated by an arrow. Fig. 18(3) shows a state in which the bellows portion of the bellows-like body fluid suction member 39 is expanded from the state shown in Fig. 18(2) to allow a body fluid, such as gastric juice, to flow in the bellows portion in the direction indicated by an arrow. Thus, by visually recognizing a color change of the indicator material 2A, the position of the catheter can be verified.
Although the member for catheter position verification in this embodiment can verify the position of a catheter only once as in the example shown in Fig. 17, the structure thereof is simple.
In the member for catheter position verification in Embodiment 14 or 15, the indicator material 2A is placed inside the body fluid suction member. However, the indicator material 2A may be provided on the surface of the member for catheter position verification, or the member for catheter position verification may be composed of a material in which the indicator material 2A is mixed. Furthermore, the suction member constituting the member for catheter position verification is not limited to the syringe 38 or the bellows-like body fluid suction member 39, and for example, the suction member may have a balloon shape.

### Brief Description of the Drawings

Fig. 1 is a diagram showing a structure in which a member 3 for catheter position verification is connected to a catheter (for enteral feeding) 3.

Fig. 2 is a diagram showing a structure in which a check valve 6 is connected between the catheter (for enteral feeding) 3 and the tubular member 2 for catheter position verification in the structure shown in Fig. 1.

Fig. 3 shows an example of a structure in which a guidewire-like member 9 for catheter position verification is incorporated into a catheter (for enteral feeding) 3.

Fig. 4 is a diagram showing a color change indicator 2 having a groove provided on one surface.

Fig. 5 is a diagram showing a color change indicator 2 having a plurality of grooves provided on one surface.

Fig. 6 is a diagram showing a color change indicator 2 having a groove on the entire one surface.

Fig. 7 is a diagram showing a color change indicator 2 having a defective portion extending over a full thickness in the center.

Fig. 8 is a diagram showing a color change indicator 2 having a porous portion that allows a liquid to pass through.

Fig. 9 is a diagram showing a button-shaped member 21 for catheter position verification and a catheter having a connecting portion 31 having a septum structure to and from which the member 21 for position verification can be connected and disconnected a plurality of times.

Fig. 10 is a schematic diagram showing a button-shaped member 21 for catheter position verification that is easily detachable.

Fig. 11 is a schematic diagram showing a structure in which a hydrophobic porous filter 27 is placed at the hole section 26 in the button-shaped member shown Fig. 10.

Fig. 12 is a schematic diagram showing the button-shaped member 21 for catheter position verification shown in Fig. 11 in which a check valve 28 is placed at the hole section 26.

Fig. 13 is a schematic diagram showing a button-shaped member 21 for catheter position verification and a connecting tube 10 which includes a connecting portion 31 having a septum structure into and by which the member 21 for position verification is inserted and held, and a check valve 28.

Fig. 14 is a schematic diagram showing a connector to be joined to a catheter, the connector including a connecting portion 31 and a male connector 33 and a female connector 34 disposed in front and rear of the connecting portion 31, the connecting portion 31 having a septum structure 32 into and by which the leg of a member for catheter position verification can be inserted and held.

Fig. 15 is a schematic diagram showing another example of a connector member in which a check valve is placed at the male member 33 of the connector shown in Fig. 14.

Fig. 16 is a diagram showing a catheter having a septum (elastic septum) 32 and an adaptor 35 which connects a storage portion 37 through the septum (elastic septum) 32, the storage portion 37 containing a plurality of pieces of an indicator material 2B.

Fig. 17 is a diagram showing an example of a member for catheter position verification in which a color change indicator material is placed in a cylinder tip 11 of a syringe 38.

Fig. 18 is a diagram showing an example of a member for catheter position verification in which a color change indicator 2 is placed in a bellows-like body fluid suction member 39.

### Reference Numerals

- 1: syringe
- 11: cylinder tip
- 2: tubular member for catheter position verification
- 2A: color change indicator
- 2B: indicator material
- 2C: leg of button-shaped member for position verification
- 21: button-shaped member for position verification
- 22: groove
- 23: fractured portion
- 24: porous structure
- 25: tubular container
- 26: vent (hole section)
- 27: hydrophobic porous filter
- 28: check valve
- 29: connection member
- 3: catheter
- 31: connecting portion of member for catheter position verification
- 32: septum (elastic septum)
- 32A: slit and/or hole formed in septum (elastic septum)
- 33: male connector
- 34: female connector
- 35: adaptor
- 36: airtight portion
- 37: storage portion
- 38: syringe
- 39: bellows-like body fluid suction member
- 4: distal end of catheter
- 5: hole in distal end of catheter
- 6: check valve
- 7: mechanism for preventing reflux
- 8: catheter-connecting portion
- 9: guidewire-like member for catheter position verification
- 91: color change indicator
- 92: proximal portion of guidewire-like member for catheter position verification
- 10: tubular container
- 101: connecting tube

### Best Mode for Carrying Out the Invention

### Description based on examples

### EXAMPLE 1

Agar (5g each) and each of the pigments shown in Table 1 were placed in each beaker, and RO water (95 mL each) was added thereto, followed by stirring under heating. The resulting mixture was spread thinly on a tray and left in a refrigerator for 2 hours. After making an appropriate cut in the resulting product, drying was performed for 24 hours with a freeze dryer to give a color change indicator (dry product). Furthermore, each of the resulting color change indicators was brought into contact with an artificial gastric juice (clear and colorless, with a pH of about 1.2) prepared by a method according to the Japanese Pharmacopoeia general test method 58, the disintegration test method. Each product showed a satisfactory color change as shown in Table 1. The amount of each pigment used shown in Table 1 corresponds to the amount of the pigment added relative to 95 mL of RO water.

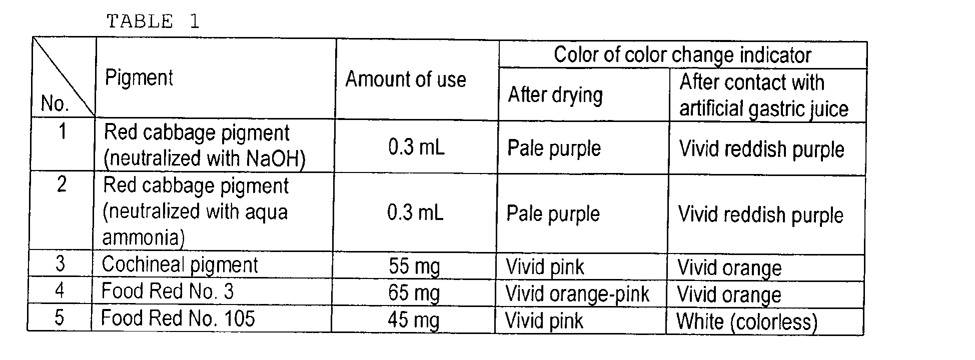

### EXAMPLE 2

The mixture obtained by stirring under heating in Example 1 was filled in a tubular container connectable to a catheter, instead of spreading thinly on the tray, and drying was performed for 24 hours with a freeze dryer. Thus, a tubular member for catheter position verification into which a color change indicator was incorporated was prepared. The color of each color change indicator after drying was the same as that shown in Table 1. Using this, a structure shown in Fig. 1 was formed. Furthermore, using the structure, insertion into the stomach was performed by the method described in Embodiment 1, and the occurrence of color change of the color change indicator due to suction of gastric juice was confirmed. Thus, it was possible to verify the arrival of the distal end of the catheter (for enteral feeding) at the inside of the stomach.

### EXAMPLE 3

A color change indicator (dry product) prepared as in Example 1 was connected to a tip of a guidewire to produce a guidewire-like member for catheter position verification. Using this, a structure shown in Fig. 3 was formed. Furthermore, using the structure, insertion into the stomach was performed by the method described in Embodiment 2, and the occurrence of color change of the color change indicator due to contact with gastric juice was confirmed. Thus, it was possible to verify the arrival of the distal end of the catheter (for enteral feeding) at the inside of the stomach.

### EXAMPLE 4

In Example 2, after verifying the arrival of the catheter (for enteral feeding) at the inside of the stomach, the tubular member for catheter position verification was replaced with a new one and connection was connection was made. Subsequently, the catheter (for enteral feeding) was inserted into the duodenum or the jejunum. (In this case, a marker showing an approximate distance to the duodenum or the jejunum was also used as an index.) At this point, the verification operation was performed as described in Embodiment 1. By verifying no color change of the color change indicator or a color change that was different from the color change upon contact with the gastric juice, it was possible to confirm that the distal end of the catheter reached the inside of the duodenum or the jejunum.

### EXAMPLE 5

In Example 3, after verifying the arrival of the catheter (for enteral feeding) at the inside of the stomach, a newly prepared guidewire-like member for catheter position verification was slowly inserted into the catheter (for enteral feeding). After confirming that the guidewire-like member for catheter position verification reached the distal end of the catheter (for enteral feeding), the catheter (for enteral feeding) was further inserted into the duodenum or the jejunum. (In this case, a marker showing an approximate distance to the duodenum or the jejunum was also used as an index.) At this point, the verification operation was performed as described in Embodiment 2. By verifying no color change of the color change indicator or a color change that was different from the color change upon contact with the gastric juice, it was possible to confirm that the distal end of the catheter (for enteral feeding) reached the inside of the duodenum or the jejunum. Industrial Applicability

The present invention provides a member for catheter position verification in which the position of a medical catheter inserted in the body, in particular, the position of the distal end region of the catheter, can be verified more simply, reliably, and safely; and a catheter including the member for catheter position verification. Consequently, it is possible to perform treatment, examination, and the like more simply, reliably, and safely using a medical catheter.

## Claims

1. A position verifying member for a catheter having permeability for body fluid, comprising: a color change indicator that changes color upon contact with a body fluid, wherein the color change indicator is to be attached to the medical catheter or a connected member connected to the catheter to be inserted in a human body.

2. The position verifying member for a catheter according to Claim 1, wherein the color change indicator comprises a pigment and a pigment carrier.

3. The position verifying member for a catheter according to Claim 1 or 2, wherein the color change indicator changes color depending on the hydrogen-ion concentration of the body fluid.

4. The position verifying member for a catheter according to Claim 3, wherein the color change indicator is adjusted to be neutral before contact with the body fluid.

5. The position verifying member for a catheter according to any one of Claims 1 to 4, wherein the color change indicator is water-soluble.

6. The position verifying member for a catheter according to any one of Claims 2 to 4, wherein the pigment is a edible pigment.

7. The position verifying member for a catheter according to any one of Claims 2 to 6, wherein the pigment carrier is composed of fibers, a structure composed of the fibers, a sponge, or a gel.

8. The position verifying member for a catheter according to Claim 7, wherein the pigment carrier is composed of a material that is safe for ingestion intake.

9. The position verifying member for a catheter according to any one of Claims 1 to 8, wherein the color change indicator is a dry product.

10. The position verifying member for a catheter according to Claim 9, wherein the pigment is an anthocyanin pigment.

11. The position verifying member for a catheter according to any one of Claims 1 to 10, wherein the color change indicator is filled in a container member.

12. The position verifying member for a catheter according to Claim 11, wherein the container member is a button-shaped member having a leg.

13. The position verifying member for a catheter according to Claim 11 or 12, further comprising a member which prevents the reflux of the body fluid that has passed through the color change indicator.

14. The position verifying member for a catheter according to any one of Claims 1 to 13, wherein the color change indicator has an easy flow structure through which the body fluid easily flows.

15. The position verifying member for a catheter according to any one of Claims 1 to 14, wherein the color change indicator is connected to an end of a guidewire.

16. A medical catheter comprising the position verifying member for a catheter according to any one of Claims 1 to 14 fitted to the catheter or a connection member connected to the catheter.

17. A medical catheter, to which a body fluid suction means is connected, wherein the position verifying member for a catheter according to any one of Claims 1 to 14 attached between the medial catheter and the body fluid suction means.

18. A medical catheter connected to a body fluid suction means via a connecting tube, wherein the position verifying member for the catheter according to any one of Claims 1 to 14 is fitted to the connecting tube.

19. A medical catheter in which the member for catheter position verification according to Claim 15 is inserted.

20. The medical catheter according to Claim 17 or 18, wherein the medical catheter has an opening at a distal end thereof, the opening allowing a body fluid to enter the catheter by means of suction by the body fluid suction means.

21. A medical catheter, comprising a sidewall, of the medical catheter, or of a connection member connected to the medical catheter, the sidewall having a connecting portion composed of a septum (elastic septum) having a slit and/or a hole (hole portion) capable of connecting the position verifying member for a catheter according to any one of Claims 1 to 14.

22. The medical catheter according to any one of Claims 16 to 21, wherein the medical catheter is a catheter (for enteral feeding).

23. A position verifying member for a catheter, comprising a syringe having a cylinder tip including a small diameter portion, wherein the position verifying member for a catheter according to any one of Claims 1 to 10 is placed in the small diameter portion.
